# EUROPEAN PATENT SPECIFICATION

(11) **EP 2 712 917 B1**
(45) Date of publication and mention of the grant of the patent: **18.11.2015**
(21) Application number: 13178678.2
(22) Date of filing: 31.07.2013
(51) Int. Cl.: C12M 1/00

(54) **Carbonation system for cultivating microalgae in open reactors**
Karbonisierungssystem zur Mikroalgenzüchtung in offenen Reaktoren
Système de carbonatation de culture de micro algues dans des réacteurs ouverts

(30) Priority: 26.09.2012 ES 201231485
(43) Date of publication of application: 02.04.2014
(73) Proprietor: FCC AQUALIA, S.A., 28016 Madrid (ES)
(72) Inventor: Lara Corona, Enrique, 41005 SEVILLA (ES); Acien Fernandez, Francisco Gabriel, 41005 SEVILLA (ES); De Godos Crespo, Ignacio, 41005 SEVILLA (ES); Mendoza Martin, José Luis, Southampton S017 1BJ United Kingdom (GB)
(74) Representative: Ungria López, Javier

(56) References cited:
- WO-A1-03/094598
- WO-A2-2007/025145
- DATABASE WPI Week 198806 Thomson Scientific, London, GB; AN 1988-038356 XP002713147, & JP S62 220183 A (SEKINE T) 28 September 1987 (1987-09-28)
- DATABASE WPI Week 201213 Thomson Scientific, London, GB; AN 2012-B81697 XP002713148, & JP 2012 023978 A (HITACHI PLANT ENG&CONSTR CO LTD) 9 February 2012 (2012-02-09)
- DATABASE WPI Week 201306 Thomson Scientific, London, GB; AN 2012-Q17160 XP002713149, & CN 102 643 741 A (CHINESE ACAD SCI PROCESS ENG INST) 22 August 2012 (2012-08-22)
- MENDOZA J L ET AL: "Fluid-dynamic characterization of real-scale raceway reactors for microalgae production", BIOMASS AND BIOENERGY, PERGAMON, AMSTERDAM, NL, vol. 54, 17 May 2013 (2013-05-17), pages 267-275, XP028567982, ISSN: 0961-9534, DOI: 10.1016/J.BIOMBIOE.2013.03.017
- DE GODOS I ET AL: "Evaluation of carbon dioxide mass transfer in raceway reactors for microalgae culture using flue gases", BIORESOURCE TECHNOLOGY, ELSEVIER BV, GB, vol. 153, 8 December 2013 (2013-12-08), pages 307-314, XP028669169, ISSN: 0960-8524, DOI: 10.1016/J.BIORTECH.2013.11.087

## Description

### Field of the invention

The present invention belongs to the field of the production of microalgae, whether for the subsequent use thereof or in liquid effluent or gas purification processes by means of their cultivation. The invention, more specifically, falls into the area of systems for carbonation or supply of CO₂ for producing microalgae in open reactors.

### State of the art

Microalgae are highly useful microorganisms as they have advantageous applications in a wide variety of area such as waste water treatment, the production of biofuels, human and animal food, the reduction of greenhouse gases or obtaining high value chemical products (Pulz and Gross, 2004). Microalgal cultures may achieve much higher levels of productivity than higher plant cultures as a result of which there is a greater fixation of CO₂ and a greater quantity of biomass produced. Furthermore, microalgal cultures require less water and do not compete with other traditional cultures since they do not require fertile land and they can use low quality water, such as waste water and brine. In the case of waste water treatment, microalgal cultures achieve a reduction in the energy consumed by the traditional active sludge systems, in addition to involving greater utilization of the nutrients contained in the waste water by means of converting it into biomass, thus avoiding its dumping and environmental problems associated therewith such as eutrophication (Munoz and Guieysse, 2006).

The microalgae production systems mainly include two different classes, open systems and closed systems. The closed systems (WO03/094598 A1) isolate the fluid from the external environment and are less exposed to its disturbances, while the open systems have greater interaction with and exposure to the environment and depend on the conditions of the environment to a greater extent. The open systems (Oswald and Golueke, 1960) have had greater success on an industrial level due to their low cost, although their use has been limited to certain extremophilic species owing to problems of contamination. However, regarding the application in waste water treatment, the control of the species in culture is not an overly determinate factor which is why this type of reactor has been the most widely used. These open reactors are known as oxidation channels or HRAP (high rate algal pond). This type of reactor, although it has been described as an example for treating waste water using microalgae, has not been suitably optimized, thus often resulting in it being limited in nutrients or in the excess accumulation of oxygen, which are factors that limit its performance.

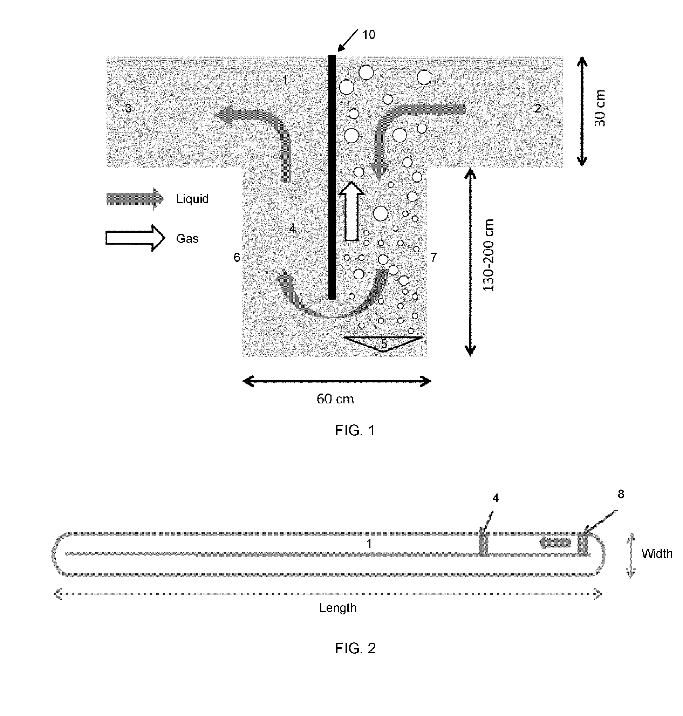

One of the most limiting factors in cultivating microalgae is the availability of carbon. It is assumed that the microalgae require 2 kg of CO₂ for each kilo of biomass produced, which is why it is necessary to supply the latter with all of this CO₂ by means of carbonation methods in order to avoid the cultures being limited in this CO₂. This gas may be supplied as pure CO₂ or as a mixture of gases. In the case of the latter, they would be flue gases having variable contents of CO₂ between 4% and 15% depending on their origin. The continuous injection of flue gases into the microalgal cultures causes them to develop in acidic conditions, reducing their performance and resulting in low use efficiencies of the CO₂, between 8.1 % (Zhang, et al., 2001) and 4.2% (Hu, et al., 1998). Injection as required, however, allows this use efficiency to be increased to 32.8% in open reactors (Doucha, et al., 2005) and 50% in closed reactors (Camacho Rubio, et al., 1999). In order to increase said use efficiency, optimized reactors with supply of CO₂ need to be designed and operated which do not involve a reemission of said CO₂ into the atmosphere (Camacho Rubio, et al., 1999). In open reactors, the gases are normally bubbled in the lower part of the reactor, which involves only a small percentage of the gas being used efficiently, the remainder dissipating into the atmosphere. In order to optimize the use efficiency of CO₂, the installation of pits, with a greater depth than that of the reactor, has been proposed, in which the gas is bubbled (Figure 1, Prior art). To maximize the use efficiency of the CO₂, the pits are fitted with a deflecting partition wall, as disclosed in JP19860063021, JP2012023978 A or CN102643741 A, which forces the water to descend to get in contact with the flow of CO₂. More than one baffle can be installed and combined with a bubble injector, as in WO 2007/025145 A2.

The design of these pits has been substantiated on the following basis of:
- Obtaining a high L:G ratio (liquid:gas). This ratio is considered fundamental for achieving a high transfer efficiency of CO₂ in water. With the help of the vertical deflector, the entire L flow is guided and effectively mixed with the gas flow G. The liquid flow L is usually very high owing to the horizontal movement produced by the mixing system as may be the case for example in a paddle wheel system, which is why this system is effective in terms of obtaining a suitable transfer ratio.
- Achieving a high water/bubble contact time: to this end, the countercurrent design was standard, as the design depicted in Fig. 1 illustrating the prior art, such that the descending current of water reduces the ascension speed of the air bubble and facilitates its dissolution. By means of this technique, it was sought to reduce the height of the pit and at the same time avoid costly public works. In other words, the dwell time of the bubble in the water is increased by the retention provoked by the water. By way of this strategy, wells with medium-sized bubbles of 3 - 5 mm were designed using injection devices which were not optimized.
- Achieving a sufficient height which increases the transfer of CO₂. Given that the height of the liquid in HRAPs is just 30 cm, which is insufficient for obtaining a high transfer efficiency, heights of between 1.3 and 2 m were considered necessary in order to obtain suitable transfers, taking the injection devices normally used into account.

However, this design has a number of significant disadvantages:
- High pit heights, close to 2 m, involve an increase in the cost of public works and problems relating to the operation;
- The deflecting partition wall is an obstacle to the flow and creates a high charge loss and therefore greater energy consumption and reduced circulation speeds;
- The deflecting partition wall eliminates the vertical speed component (waves) which generate the mixing system. This vertical component is considered important for a high level of productivity of the algae in the reactor, as it promotes the dispersion of the liquid medium which is directly related to the exposure of the microalgae to light; and
- It makes the use of flue gases in large-area HRAPs difficult. In order to reduce the cost of industrial installations, it is important to construct reactors of a high unit size, above 3000-4000 m²/Ud, thus reducing the installation of tubing, and elements for the control, mixing and injection of CO₂. An example would be designing a reactor being 10,000 m² (W=20 m x L=500 m) having a single pit being 10 m in width continuing from the mixing system (Figure 2) and having a maximum level of algal productivity of 4 g/m²/h. If flue gases with a 10% CO₂ content are injected, flows of 450 m³/h are required to cover the CO₂ demand (10 g of CO₂/m²/h). In a pit measuring 10x0.3, this would involve a flow of 700 I of gases/m from contactor/min and an approximate gas volume value of 25% with respect to the liquid. This value is excessively higher than the normal acceptable value for obtaining a high gas/liquid transfer (<10%; Sha, 1982). The limitation on the injection of gases into the countercurrent is based on the small number of pits having a deflector which may be installed in these types of reactor (the greater the number of pits, the lower the speed of circulation). According to the prior art, large installations, therefore, have a single pit.

To summarize, the use of a current of flue gases as a source of CO₂ in a pit such as those used normally, would produce an excess gas flow per volume of the exchanger giving rise to bubble coalescence phenomena and a reduction in the CO₂ transfer efficiency. In order to avoid this problem, installing more than one pit could be envisaged, however, this would lead to problems relating to cost, energy consumption and would make the movement of liquid along the HRAP difficult owing to the presence of the deflecting partition wall, which is why this is not a valid option on an industrial scale.

In order to solve the problems indentified in the prior art, the subject matter of the invention is an optimized system for supplying CO₂ (carbonation) to microalgal cultures contained within an open reactor, allowing for the transfer of CO₂ required by the microalgal cultures in order for them to grow in an efficient manner, using any gas containing CO₂, whether a flow of pure CO₂ or flue gases with a variable content of CO₂. The device supplying CO₂ to the open reactor, which may, in particular, be an open, mixed pond reactor of the raceway or HRAP type, ensures a controlled supply of CO₂ to the microalgal cultures, preventing them becoming limited in their productivity due to a deficient source of CO₂, while also optimizing the production by being able to keep stable the pH of the culture medium. The device supplying CO₂, that is a fine bubble diffuser selected from a fine bubble membrane or a ceramic diffuser, optimizes the purification of the gas used as a source of CO₂, adjusting its flow and the design of the supply system to achieve a high gas purification and therefore, a greater utilization of the CO₂ supplied, with minimum energy consumption. The optimum performance of the system has been achieved by means of its suitable design (dimensions and number of pits and the sparger used) and operation (liquid and gas flow).

### General description of the invention

In order to resolve the problems in the current design of facilities with supply of CO₂ gas to microalgal cultures such as those contained in open reactors and, in particular, those in the mixed pond, raceway or HRAP type, the present invention proposes the design of a novel system configured to optimize said supply and the growth of the cultures. Accordingly, the present invention provides a carbonation system for open reactors as described in claim 1.

The invention, thus, relates to a carbonation system, (supply of a flow of gas containing CO₂) for cultivating microalgae in an open reactor comprising:
- at least one flow channel (1) through which a current of water flows, in which the microalgae are cultivated, provided with an inlet (2) and an outlet (3), and
- at least one pit (4) without a deflecting partition wall located at the bottom of the flow channel (1),
said system being **characterized in that** the pit has a width equal to the width of the flow channel and a length between half to double the width of the channel; and in that the pit comprises one or a plurality of gas injection devices (5), that are fine bubble membranes or ceramic diffusers, for injecting the gas flow into the interior of the pit from the bottom to the top side, the gas injection device (5) being located at the base (bottom) of said pit and adjacent to the wall (6) opposite to the inlet direction of the water current, and at a height such that the top side of the injection device (5) through which the gas is vertically injected is at a distance of between 0.5 and 1.5 m, both limits included, from the bottom of the flow channel (1), which is to say, from the upper opening of the pit (4) (where the latter joins the bottom of the channel).

The carbonation system or CO₂ supply is essentially integrated into the open reactor having a flow channel (1) through which the sheet of water in which the microalgae are cultivated flows, and one or a plurality of pits (4) at the bottom or base of said channel (1), having the shape and dimensions as those defined and smaller than those normally used in these types of facilities, in which the injection devices (5) of gas into the interior of the pit (4) are arranged, such that said gas injectors (5) are located specifically at the base or bottom of the pit (4) and adjacent to the vertical wall (6) of the pit which is located at the side opposite to the inlet of the current of water into the channel (1). In this manner, the injectors project the current of gas containing the CO₂ from below (bottom of the pit (4)) in an upwards direction (channel (1) of water), causing the optimum mixing of both fluid.

The relevant aspect is constituted by the distance from the top side of the injection device (5) through which the gas enters into the interior of the pit (4) up to the bottom of the flow channel (1), which is to say, the upper opening of the pit (4) (where the pit and flow channel (1) join) since it is the distance covered by the gas bubble and therefore, defines the contact time and as a result the transfer. This distance must be between 0.5 and 1.5 m.

When it is confirmed that the injection device (5) is adjacent to the wall (6), it must be understood that it is at a sufficient distance for driving the gas towards the bottom of the flow channel (1), that is to say, vertically and in a suitable manner such that the proximity to the wall (6) does not cause a change in the dynamic of the gas flow which interferes with the transfer thereof into the liquid. In practice, this proximity may, preferably, be between 50 and 150 mm, both limits included.

The distance of the gas injection device (5) from the bottom of the pit (4) is determined only by matters of design such as the geometry of the diffuser (5), its size, diameter of the tubing for supplying gas, the distance at which the diffuser (5) is arranged from bottom, etc. Taking these design matters into account, from a design point of view, it would most suitable for the distance of the diffuser (5) from the bottom of the pit (4) to be as small as possible, for reasons of design economy and because this would produce a "dead" volume under the mixing produced by the diffuser (5), with potential decanting problems and problems concerning the fermentation of the algal biomass.

Open reactors, in particular those of the mixed pond, raceway or HRAP type, consist of two or four channels open to the atmosphere, of one surface between 100 and 10,000 m² and a liquid height of between 0.2 and 0.4 m, by means of which the microalgal culture is recirculated by way of mechanical systems such as pumps or paddle wheels. These type of reactors are the most widely used internationally for producing microalgae, however, they have disadvantages in terms of their material transfer capacity, in particular CO₂.

It must be understood that the flow channel (1), through which the water current in which the microalgae are cultivated flows, is designed conventionally, in a manner known in the field. It, thus, has a flat bottom, on which the pits (4) are located, and lateral walls delimiting the interior of the channel and, therefore, the volume of the current of water.

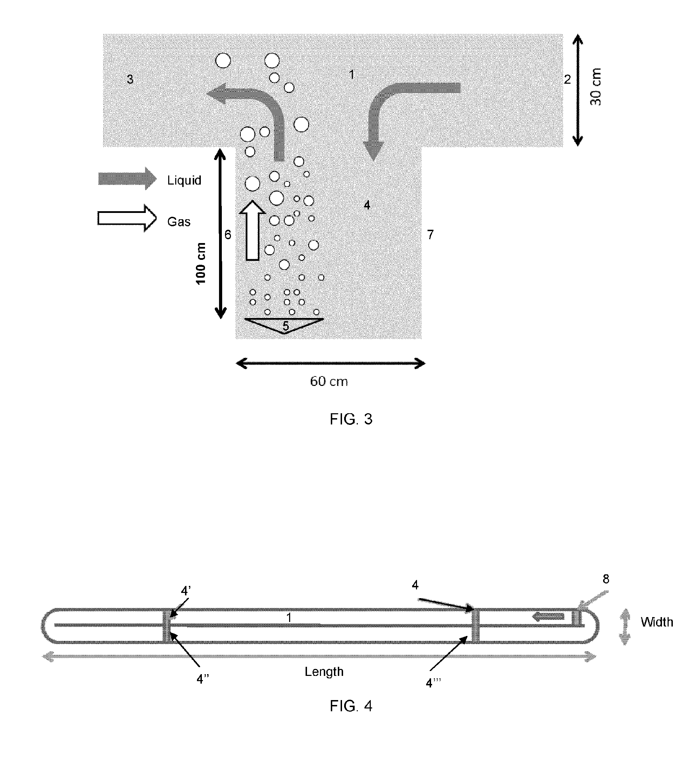

Figure 3 shows a schematic illustration of the system described above, which must not be considered as limiting the system in all its aspects, but merely in the essential configuration described (for example the lengths in the figure are guideline values). It can be seen in this design that the current of liquid L flows through the flow channel (1) and is mixed with the current of gas G without the help of a deflector. In this fundamental design, the injection of gas is carried out by means of a diffuser (5) arranged closer to the vertical wall (6), opposite to the direction of L, than its opposite to vertical wall (7). The diffuser (5) is specifically at a distance of between 50 and 150 mm from the vertical wall (6) opposite to the flow of the liquid L. In this way, part of the current L is forced to descend vertically through the pit (4) in order to engage the ascending pump flow generated by the gas and promoting the mixing of L and G. This mixing of the two currents, liquid and gas, is considered essential for a high transfer of CO₂ in the liquid.

The system which is the subject matter of the invention has various advantages over other systems known from the prior art, in various aspects which emerge from its design and performance.
1. Absence of deflecting partition wall: By removing the deflecting partition wall, the problems arising from the arrangement thereof are avoided, such as greater energy consumption and the reduction of cell exposure to the light.
2. Use of fine bubble diffusers such as CO₂ injector devices: the use of these fine bubble diffusers, being membrane or ceramic diffusers, normally used in waste water purification systems for injecting oxygen, allowed an 99% CO₂ transfer efficiency to be obtained with respect to other gas supply systems by mean of which maximum values of 60% are achieved. They are also very economic.
3. Operating with high L:G ratios: the ratio of L:G in the contact system determines the purification of the gas and therefore the efficiency of the system, which is why this must be as high as possible in order to maximize gas purification, however, without incurring excess energy consumption. The most suitable values for the L:G ratio are between 10 and 50 cm/sg. A suitable contact between the phases is necessary, which is one of the principal challenges overcome by the present invention, specifically, by having focused research into the design of the system on the following points:
   - defining the minimum interval of the liquid flow, L, below which suitable dissolutions of the gas are not obtained, and the maximum, above which the consumption of energy is excessive. This range defined the design of the mixing element or the mixing device, such as for example a paddle wheel. In this configuration of the system which is the object of the invention, the minimum flow is significant and above that which is obtained using a deflecting partition wall, due to the fact that the L:G mixture is not 'perfect', as in the last case. A greater liquid flow was thus necessary for achieving the same performances as with the deflecting partition wall. It was checked that the minimum flow for achieving transfer performances above 95% were in the normal functioning intervals for mixing systems, such as the paddle wheel (> 25 cm/s liquid speed). Therefore, the system proposed did not have a drawback in this respect. In addition, it must also be taken into account that removing the deflecting partition wall enabled operation at greater speeds of the liquid, making use of a smaller amount of energy.
   - Defining the minimum interval of the gas flow, G, below which the effect of mixing with the current of liquid was insufficient, and the maximum proceeding from which the bubble coalescence phenomena or increase in the size of the bubble were produced. These phenomena of excessive flow caused a reduction in the CO₂ transfer efficiency.
   - Defining the number of pits required, which in its most fundamental form is a single pit, the optimization of which was carried out as a function of the level of productivity of the biomass desired and the CO₂ transfer capacity of the pit. Taking into account that the CO₂ requirements are approximately 2 kg of CO₂ per 1 kg of biomass produced, the optimum number of pits was obtained as the quotient between the CO₂ consumption and the CO₂ transfer rate in each pit.
4. Shallow pit. The decrease in the size of the pit with respect to the known systems in the field involves a reduction in the cost of the construction and of public works. The height of the pit is less than the 2 m of the pits normally used and has been reduced to a minimum of 0.5 m, although this must be determined on a case-by-case basis depending on the overall design of the reactor and its size.
5. Optimum number of pits. Not having the drawback of energy consumption when increasing the number of pits per reactor, allowed the number of pits to be increased compared with the prior art. However, as has been mentioned, the number of pits must be optimized as a function of the level of productivity of the biomass or CO₂ consumption rate and the CO₂ transfer rate in the pit, as well as its distribution as a function of the final design of the reactor and the level of productivity reached. A plurality of optimized pits has the following advantages:
   - Optimization of the injection of the gas flow per linear meter of the pit, for the transfer criteria and not conditioned by a maximum gas flow to be injected.
   - Oxygen desorption in each one of the pits, by means of which an oxygen level lower than that throughout the reactor is maintained, reducing photooxidation and photorespiration phenomena and resulting in an increase to the level of productivity of the microalgal cultures. This is another of the criteria to take into account for the design of a plurality of pits.
   - Minimum variations to the pH throughout the reactor, without excessive increases, by means of which a greater performance of the algal cultures would be obtained by being subjected to less stress. This pH increase is another of the criteria for calculating the number of pits.

A second object of the present invention is a carbonation method (supplying a gas flow containing CO₂) in an open reactor for cultivating microalgae as described in claim 12, by means of using the carbonation system described above and which comprises the step of injecting the gas flow containing the CO₂ into the interior of the pit (4) without a deflecting partition wall as defined before, by means of the gas injection device (5) located at the base thereof adjacent to the wall (6) opposite to the inlet of the current of water and at a height such that the top side of said device (5) is at a distance of between 0.5 and 1.5 m from the bottom of the flow channel (1), in a CO₂ volume of between 0.008 and 0.025 v/v/min (volume of CO₂/volume of pit/min), both limits included, maintaining the circulation speed of the liquid at between 0.1 and 0.5 m/s inclusive, which is equal to a liquid:gas (L:G) ratio in the pit (4) above 10. As has been mentioned, this method, compared to other known systems, achieves the mixing of the current of liquid L flowing though the flow channel (1) with the current of gas G without the help of a deflector, in such a way that owing to the arrangement of the injection device (5) at the bottom of the pit (4), the mixing of the gas over the water (water pumping owing to the lower density combination of gas-liquid) is promoted. In this way, part of the current L is forced to descend vertically through the pit (4), to engage the ascending pump flow generated by the gas and promoting the mixing of L and G.

A third subject matter of the present invention is applying the system disclosed here for one of the following uses:
- waste water treatment, using mixed, microalgae-bacteria systems, as described in claim 14;
- algae production, for energy and food purposes, as described in claim 15;
- production of high added value compounds such as antioxidants, phycobiliproteins, products with biological activity;
- eliminating greenhouse gases contained in the current of gas injected as the source of CO₂; and
- purification of industrial gases containing CO₂ which are injected into the system, both flue gases as well as biogas, by means of absorption of the contaminants present therein and subsequent biological fixation by means of microalgae production, as described in claim 16.

### Detailed description of the invention

In one particular embodiment of the invention, the flow of carbonating gas is a flow of pure CO₂. In another preferred embodiment, the current of gas consists of flue gases, that is to say, produced in a combustion process. In this case, the current of gas preferably contains a percentage of CO₂ of between 4% and 20%, more preferably between 8% and 11%, both limits included.

The reactor or open system in which the microalgae are cultivated is preferably a rapid flow channel, also called a raceway or HRAP type reactor. In a particular embodiment of the invention, the system comprises 2 to 4 flow channels.

The pit (4) preferably has a depth and a length of between 0.5 and 1.5 m, that is to say, the top side of the injection device (5) is arranged precisely at the base or bottom of the pit (4), since this is the required distance for suitable diffusion which must be observed with respect to the bottom of the flow channel (1). The depth and length of the pit (4) are more preferably between 0.5 m and 1 m, both limits included, with 1 m being more preferred.

The number of pits (4) in the carbonation system by supplying CO₂ may be adjusted depending on the size of the reactor. The system thus preferably comprises a defined number of pits (4) distributed uniformly on the surface of the reactor and which overall, meet the CO₂ demands of the culture.

As disclosed above, the gas injection device (5) is a fine bubble membrane or ceramic diffuser. These diffusers are usually used in other systems, such as in those for waste water purification and their configuration is known in the field.

The system may incorporate a water mixing device (8) in the channel (1) which may, most preferably, be a paddle wheel device.

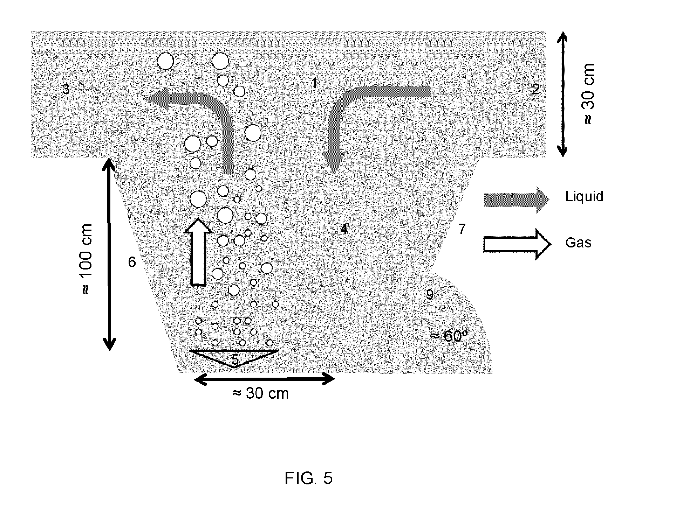

In the most preferred embodiment of the invention, illustrated in Figure 5, the hydraulics in the essential design of the system are optimized, removing dead areas at the bottom of the pit (4) (and thus avoiding possible decanting of the culture) and facilitating the outlet and inlet of the water flow in the reactor which allows the charge losses in the reactor to be minimized. In order to remove the dead areas and decanting of solids at the bottom of the pit (4), this system is constructed with walls (6, 7) inclined towards both sides of the diffuser (5) between 45° and 75° to the horizontal, in such a way that the base of the pit (4) has a smaller length with respect to its upper part in contact with the channel (1). The bottom of the pit (4), therefore, has reduced dimensions compared to the previous embodiment in Figure 3. Specifically, it is sufficient to leave a 200 - 400 mm space which is required for the arrangement of the diffuser (5). In this way, it is possible for the solids to decant towards the area where the diffusers (5) are installed and allows them to be maintained in suspension. At the same time, the walls (6, 7) inclined towards both sides of the diffusers (5) improve the mixture, contributing a certain element of vertical flow both at the inlet of the pit (4) and at the outlet, with respect to the previously horizontal flow generated by the paddle wheel (8). In order to facilitate the inlet and outlet of water to/from the channel (1), gases are injected into a flow rate in the range of 0.008 to 0.025 v/v/min (CO₂ volume). In a preferred embodiment, the wall (7) of the pit (4) closest to the inlet (2) of water to the channel (1) has an interior concave (9).

With respect to the method for supplying CO₂, the liquid:gas ratio is between 0.1 and 0.5 m/sg (10 and 50 cm/sg), although preferably, it is between 20 and 30 cm/sg, both limits included.

### Description of the figures

**Figure 1****.** A traversal view of the carbonation system by supplying CO₂ usually used in open reactors with a deflecting partition wall inside the reactor (prior art).
   (1) Flow channel
   (2) Inlet of water to the flow channel
   (3) Outlet of water from the flow channel
   (4) Pit
   (5) Injector of gas to the interior of the pit (4)
   (6) Vertical wall of the pit (4) opposite to the inlet (2) of the flow channel (1)
   (7) Vertical wall of the pit (4) opposite the wall (6) and closer to the inlet (2) of the flow channel (1)
   (10) Deflecting partition wall
**Figure 2****.** Plan view of an open reactor of the raceway type with a pit supplied with CO₂ (Prior art).
   (1) Flow channel
   (4) Pit
   (8) Flow mixing device
**Figure 3****.** Transversal view of an illustrative example of the carbonation system by supplying CO₂ which is the subject matter of the present invention.
   (1) Flow channel
   (2) Inlet of water to the flow channel
   (3) Outlet of water from the flow channel
   (4) Pit
   (5) Injector of gas to the interior of the pit (4)
   (6) Vertical wall of the pit (4) opposite to the inlet (2) of the flow channel (1)
   (7) Vertical wall of the pit (4) opposite the wall (6) and closer to the inlet (2) of the flow channel (1)
**Figure 4****.** Plan view of an illustrative example of the design proposed by the present invention of a raceway type reactor having a plurality of pits supplied with CO₂ described in Example 1.
   (1) Water flow channel
   (4) First pit
   (4') Second pit
   (4") Third pit
   (4"') Fourth pit
   (5) Mixing device - paddle wheel
**Figure 5****.** Transversal view of a carbonation system by supplying CO₂ optimized according to the present invention. The wall of the pit located at the inlet side of the water flow to the channel has a concave shape, preventing the occurrence of dead areas and the accumulation of culture.
   (1) Flow channel
   (2) Inlet of water to the flow channel
   (3) Outlet of water from the flow channel
   (4) Pit
   (5) Injector of gas to the interior of the pit (4)
   (6) Vertical wall of the pit (4) opposite to the inlet (2) of the flow channel (1)
   (7) Vertical wall of the pit (4) opposite the other vertical wall (6) and closer to the inlet (2) of the flow channel (1)
   (9) Interior concave of the wall (7) of the pit (4) close to the inlet (2) of the flow channel (1).

### Example 1. Carbonation pit of a system for cultivating microalgae of the raceway type using flue gases according to the present invention.

Various carbonation tests were carried out in a pit (4) such as that described in Figures 2 and 3 in a 100 m² raceway reactor. The pit (4) was integrated into the cultivating system, causing a continuous exchange between the liquid residing in the pit (4) and the culture medium present in the entire system owing to the continuous mixing which is normally carried in open microalgal reactors. The width of the pit (4) was 0.6 m and the depth 1 m, without taking into account the depth of the culture. The width of the pit (4) constructed in this manner was in the order of twice the depth of the culture in the raceway (0.3 m) in order to promote the liquid exchange in the contactor. The length of the pit (4) was equal to the width of the channel (1), this being 1 m. The injection devices (5) were fine bubble discs of 270 mm, constructed with a circular EPDM membrane and with around 6,600 slots/diffuser. Said diffusers (5) were arranged at approximately 10 cm from the last wall (6) and distributed along the width of the pit (4) preventing bubble coalescence phenomena. Three diffusers (5) were arranged at the bottom of the pit (4), thus, three diffusers (6) per lateral meter.

Under optimal operating conditions, this system made it possible to introduce into the culture sufficient CO₂ in order to satisfy the growth of the microalgae. In the experiment, it was checked that said conditions were reached when the L:G ratios were maintained at values of between 10 and 50 cm/sg. The liquid flow rate was determined by the mixing system (8), which normally operates at speeds of between 0.1 and 0.3 m/s (flow rate of 20 - 60 L/s, at a depth of 0.2 m) and the gas flow rate is determined by the conditions of maximum productivity: above 0.8 - 1.5 L/m² min (1-1.6 L/s). The guaranteed maximum transfer (> 95%) is achieved when the exchanger operates with flows within the ranges established and for the configuration described in this example.

In the system previously described, i.e. the 100 m² raceway provided with a pit (4), transfer performances above 95% were reached. In contrast to the carbonation systems previously described in the prior art, in which lower performances (50-80%, Weissman et al., 1989) were reached and said efficiency was unverified, in this system the quantity of CO₂ transferred by means of material balances established in both phases was checked: elimination of CO₂ from the gases injected into the pit (4) and increase of CO₂ in the liquid in circulation. In addition, the system was verified by means of mass balances established in the actual microalgal cultures. In these cultures, high recoveries of carbon injected as a source of CO₂ was found in the form of biomass (> 90%) and the stoichiometric balances indicated for this type of microorganism were verified: approximately 2 grams of CO₂ per gram of biomass produced. This last piece of data verifies the carbonation system and presents an advantage over the other systems described in the scientific literature in which the transfer efficiency was only verified in terms of chemical transfer without verifying the actual conditions of the culture (Putt et al., 2010). The cultures tested using this carbonation system reached productivity levels of biomass close to the maximums described for this type of system: up to 22 g/m⁻² d⁻¹.

### References

Camacho Rubio F, Acien Fernández FG, Sánchez Pérez JA, Garcia Camacho F, Molina Grima E. 1999. Prediction of dissolved oxygen and carbon dioxide concentration profiles in tubular photobioreactors for microalgal culture. Biotechnology and Bioengineering 62:71-86.
Doucha J, Straka F, Lívanský K. 2005. Utilization of flue gas for cultivation of microalgae (Chlorella sp.) in an outdoor open thin-layer photobioreactor. Journal of Applied Phycology 17:403-412.
Hu Q, Kurano N, Kawachi M, Iwasaki I, Miyachi S. 1998. Ultrahigh-cell-density culture of a marine green alga Chlorococcum littorale in a flat-plate photobioreactor. Appl Microbiol Biotechnol 49:655-662.
Muñoz R, Guieysse B. 2006. Algal-bacterial processes for the treatment of hazardous contaminants: A review. Water Res 40:2799-2815.
Oswald WJ, Golueke CG. 1960. Biological transformation of solar energy. Adv Appl Microbiol 2:223-262.
Pulz O, Gross W. 2004. Valuable products from biotechnology of microalgae. Applied Microbiology and Biotechnology 65:635-648.
Singh M, Chinnasamy S, Das KC. An efficient system for carbonation of high-rate algae pond water to enhance CO2 mass transfer. Bioresour Technol. 2011 Feb;102(3):3240-5. 2010.
Zhang K, Miyachi S, Kurano N. 2001. Photosynthetic performance of a cyanobacterium in a vertical flat-plate photobioreactor for outdoor microalgal production and fixation of CO2. Biotechnology Letters 23:21-26.
Shah, Y., Kelkar, B., Delker, W., 1982. Design parameters estimations for bubble. Column reactors. AIChE J. 28 (3), 353-379.
Weissmann JC, Tillet, DM, Goebel, RP. 1989. Design and operation of an outdoor microalgae test facility. Final subcontract report. SERI Technical Monitor: W. Bollmeier. U.S Department of Energy.

## Claims

1. A carbonation system by supplying a gas flow containing CO₂ in an open reactor for cultivating microalgae comprising:
- at least one flow channel (1) through which a current of water flows, in which the microalgae are cultivated, provided with an inlet (2) and an outlet (3), and
- at least one pit (4) without a deflecting partition wall, located at the bottom of the flow channel (1),
said system being **characterized in that** the pit (4) has a width equal to the width of the flow channel (1) and a length between half to double the width of the channel (1); and **in that** the pit comprises at least one gas injection device (5) that is a fine bubble membrane or ceramic diffuser for injecting the gas flow from the bottom to the top side of the pit (4), the gas injection device (5) being located at the base of said pit and adjacent to the wall (6) opposite to the inlet direction of the water current; the distance between the top side of the injection device (5), through which the gas is vertically injected, and the bottom of the flow channel (1) being of between 0.5 and 1.5 m, both limits included.

2. The carbonation system according to the preceding claim, wherein the gas flow is a flow of pure CO₂.

3. The carbonation system according to claim 1, wherein the gas flow is a flow of flue gases.

4. The carbonation system according to claim 3, wherein the gas flow contains a percentage of CO₂ of between 4% and 20%, both limits included.

5. The carbonation system according to any one of claims 1 to 4, which is a rapid channel flow system or a high rate algal pond reactor.

6. The carbonation system according to any one of claims 1 to 5, wherein the pit (4) has a depth and a length of between 0.5 m and 1 m, both limits included.

7. The carbonation system according to any one of claims 1 to 6, wherein the gas injection device (5) is located at a distance of between 50 and 150 mm from the wall (6) opposite the inlet direction of the water current.

8. The carbonation system according to any one of claims 1 to 7, which comprises, at the bottom of each pit (4), three gas injection devices (5) per meter of width of the pit.

9. The carbonation system according to any one of claims 1 to 8, which comprises a water mixing device (8) in the channel (1).

10. The carbonation system according to any one of claims 1 to 9, wherein the wall (7) of the pit (4) closer to the water inlet (2) to the channel (1) has an interior concave (9).

11. The carbonation system according to any one of claims 1 to 10, wherein the walls (6, 7) of the pit (4) located at both sides of the injection device (5) are inclined at between 45° and 75° with respect to the horizontal.

12. A carbonation method for supplying a gas flow containing CO₂ to the interior of an open reactor for cultivating microalgae by using the carbonation system described in any one of claims 1 to 11, **characterized in that** it comprises the step of injecting the gas flow containing CO₂ into the interior of the pit (4) without a deflecting partition wall as defined in claim 1, by means of the injection device (5) located at the base of said pit adjacent to the wall (6) opposite the inlet (2) of the water current and at a height such that the top side of the injection device (5) is at a distance of between 0.5 and 1.5 m from the bottom of the flow channel (1), maintaining the gas flow rate in a volumetric flow of CO₂ of between 0.008 and 0.025 v/v/min and a circulation speed of liquid of between 10 and 50 cm/s, until reaching a flow rate ratio of liquid:gas above 10.

13. The carbonation method according to the preceding claim, wherein the liquid:gas ratio is between 20 and 30 cm/sg.

14. Use of the carbonation system described in any one of claims 1 to 11 for the treatment of water waste by means of mixed, microalgae-bacteria systems.

15. Use of the carbonation system described in any one of claims 1 to 11 for the production of algae for energy and food purposes or for the production of high energy compounds.

16. Use of the carbonation system described in any one of claims 1 to 11 for the purification of industrial gases by means of absorption of the contaminants present in the gases and subsequent biological fixation by means of microalgae production.

## Patentansprüche

1. Karbonisierungssystem durch Zufuhr eines CO₂-haltigen Gasstromes in einen offenen Reaktor zum Kultivieren von Mikroalgen, umfassend:
- wenigstens einen Strömungskanal (1), durch welchen ein Wasserstrom fließt, in welchen die Mikroalgen kultiviert werden, bereitgestellt mit einem Einlass (2) und einem Auslass (3), und
- wenigstens einer Grube (4) ohne eine ablenkende Trennwand, die an dem Boden des Strömungskanals (1) angeordnet ist,
wobei das System **dadurch gekennzeichnet ist, dass** die Grube (4) eine Breite aufweist, die der Breite des Strömungskanals (1) entspricht und eine Länge zwischen der halben bis doppelten Breite des Kanals (1); und dass die Grube wenigstens eine Gasinjektionseinrichtung (5) umfasst, die eine feine Blasenmembran oder ein keramischer Diffusor ist, um den Gasstrom von dem Boden zu der Oberseite der Grube (4) zu injizieren, die Gasinjektionseinrichtung (5) an der Basis der Grube angeordnet ist und benachbart zu der Wand (6), welche der Einlassrichtung des Wasserstroms gegenüberliegt; wobei der Abstand zwischen der Oberseite der Injektionseinrichtung (5), durch welche das Gas vertikal eingeblasen wird, und dem Boden des Strömungskanals (1) zwischen 0,5 und 1,5 m beträgt, wobei die Grenzwerte umfasst sind.

2. Karbonisierungssystem nach dem vorangehenden Anspruch, wobei der Gasstrom ein Strom aus reinem CO₂ ist.

3. Karbonisierungssystem nach Anspruch 1, wobei der Gasstrom ein Rauchgasstrom ist.

4. Karbonisierungssystem nach Anspruch 3, wobei der Gasstrom einen Prozentanteil an CO₂ von zwischen 4% und 20% enthält, wobei die Grenzwerte umfasst sind.

5. Karbonisierungssystem nach einem der Ansprüche 1 bis 4, welcher ein schnelles Strömungskanalsystem oder ein High Rate Algal Pond Reaktor ist.

6. Karbonisierungssystem nach einem der Ansprüche 1 bis 5, wobei die Grube (4) eine Tiefe und eine Länge von zwischen 0,5 m und 1 m umfasst, wobei die Grenzwerte einschlossen sind.

7. Karbonisierungssystem nach einem der Ansprüche 1 bis 6, wobei die Gasinjektionseinrichtung (5) unter einem Abstand von zwischen 50 und 150 mm von der Wand (6) angeordnet ist, die der Einlassrichtung des Wasserstroms gegenüberliegt.

8. Karbonisierungssystem nach einem der Ansprüche 1 bis 7, umfassend an dem Boden jeder Grube (4) drei Gasinjektionseinrichtungen (5) je Meter der Breite der Grube.

9. Karbonisierungssystem nach einem der Ansprüche 1 bis 8, umfassend eine Wassermischeinrichtung (8) in dem Kanal (1).

10. Karbonisierungssystem nach einem der Ansprüche 1 bis 9, wobei die Wand (7) der Grube (4), welche dem Wassereinlass (2) zu dem Kanal (1) näher ist, eine innere Wölbung (9) aufweist.

11. Karbonisierungssystem nach einem der Ansprüche 1 bis 10, wobei die Wände (6, 7) der Grube (4), welche an beiden Seiten der Injektionseinrichtung (5) angeordnet sind, mit zwischen 45 und 75° in Bezug auf die Horizontale geneigt sind.

12. Karbonisierungsverfahren zum Zuführen eines CO₂-haltigen Gasstromes in das Innere eines offenen Reaktors zum Kultivieren von Mikroalgen unter Verwendung des Karbonisierungssystems, welches in einem der Ansprüche 1 bis 11 beschrieben ist, **dadurch gekennzeichnet, dass** es den Schritt des Injizierens eines CO₂-haltigen Gasstromes in das Innere der Grube (4), welche keine ablenkende Trennwand aufweist, wie in Anspruch 1 definiert, umfasst, mittels der an der Basis der Grube in der Nähe der Wand (6), welche dem Einlass (2) des Wasserstroms gegenüberliegt, mit einer Höhe angeordneten Injektionseinrichtung (5), so dass die Oberseite der Injektionseinrichtung (5) unter einem Abstand von zwischen 0,5 und 1,5 m von dem Boden des Strömungskanals (1) angeordnet ist, wobei die Gasströmungsgeschwindigkeit in einem volumentrischen Fluss von CO₂ von zwischen 0,008 und 0,025 V/V/min gehalten wird und bei einer Zirkulationsgeschwindigkeit der Flüssigkeit von zwischen 10 und 50 cm/s, bis ein Strömungsgeschwindigkeitsverhältnis der Flüssigkeit:Gas von oberhalb 10 erzielt wird.

13. Karbonisierungsverfahren nach dem vorangehenden Anspruch, wobei das Flüssigkeit:Gas Verhältnis zwischen 20 und 30 cm/sg liegt.

14. Verwendung des Karbonisierungssystems, welches in einem der Ansprüche 1 bis 11 beschrieben ist, zur Behandlung von Abwasser unter Verwendung von gemischten Mikroalgen/Bakteriensystemen.

15. Verwendung des Karbonisierungssystems, welches in einem der Ansprüche 1 bis 11 beschrieben ist, zur Herstellung von Algen zu Energie- und Nahrungsmittelzwecken oder zur Herstellung von hochenergetischen Verbindungen.

16. Verwendung des Karbonisierungssystems, welches in einem der Ansprüche 1 bis 11 beschrieben ist, zur Reinigung der industriellen Gase mittels Absorption der in den Gasen vorhandenen Verunreinigungen und nachfolgende biologische Fixierung mittels der Mikroalgenherstellung.

## Revendications

1. Système de carbonatation en fournissant un écoulement de gaz contenant du CO₂ dans un réacteur ouvert pour cultiver des micro-algues comprenant :
- au moins un canal d'écoulement (1) à travers lequel un courant d'eau s'écoule, dans lequel les micro-algues sont cultivées, pourvu d'une entrée (2) et d'une sortie (3), et
- au moins un puits (4) sans paroi de séparation défléchissante, situé dans le fond du canal d'écoulement (1),
ledit système étant **caractérisé en ce que** le puits (4) a une largeur égale à la largeur du canal d'écoulement (1) et une longueur entre une moitié et le double de la largeur du canal (1) ; et **en ce que** le puits comprend au moins un dispositif d'injection de gaz (5) qui est une membrane ou un diffuseur en céramique de fines bulles pour injecter l'écoulement de gaz du côté inférieur vers le côté supérieur du puits (4), le dispositif d'injection de gaz (5) étant situé au niveau de la base dudit puits et adjacent à la paroi (6) opposée à la direction d'entrée du courant d'eau ; la distance entre le côté supérieur du dispositif d'injection (5), à travers lequel le gaz est injecté verticalement, et le côté inférieur du canal d'écoulement (1) étant entre 0,5 et 1,5 m, les deux limites étant incluses.

2. Système de carbonatation selon la revendication précédente, dans lequel l'écoulement de gaz est un écoulement de CO₂ pur.

3. Système de carbonatation selon la revendication 1, dans lequel l'écoulement de gaz est un écoulement de gaz de combustion.

4. Système de carbonatation selon la revendication 3, dans lequel l'écoulement de gaz contient un pourcentage de CO₂ entre 4 % et 20 %, les deux limites étant incluses.

5. Système de carbonatation selon l'une quelconque des revendications 1 à 4, qui est un système d'écoulement à canaux rapide ou un réacteur bassin d'algues à haut débit.

6. Système de carbonatation selon l'une quelconque des revendications 1 à 5, dans lequel le puits (4) a une profondeur et une longueur entre 0,5 m et 1 m, les deux limites étant incluses.

7. Système de carbonatation selon l'une quelconque des revendications 1 à 6, dans lequel le dispositif d'injection de gaz (5) est situé à une distance entre 50 et 150 mm de la paroi (6) opposée à la direction d'entrée du courant d'eau.

8. Système de carbonatation selon l'une quelconque des revendications 1 à 7, qui comprend, dans le fond de chaque puits (4), trois dispositifs d'injection de gaz (5) par mètre de largeur du puits.

9. Système de carbonatation selon l'une quelconque des revendications 1 à 8, qui comprend un dispositif de mélange d'eau (8) dans le canal (1).

10. Système de carbonatation selon l'une quelconque des revendications 1 à 9, dans lequel la paroi (7) du puits (4) plus près de l'entrée d'eau (2) vers le canal (1) a une partie concave intérieure (9).

11. Système de carbonatation selon l'une quelconque des revendications 1 à 10, dans lequel les parois (6, 7) du puits (4) situées des deux côtés du dispositif d'injection (5) sont inclinées entre 45° et 75° par rapport à l'horizontale.

12. Procédé de carbonatation pour fournir un écoulement de gaz contenant du CO₂ à l'intérieur d'un réacteur ouvert pour cultiver des micro-algues en utilisant le système de carbonatation décrit selon l'une quelconque des revendications 1 à 11, **caractérisé en ce qu'**il comprend l'étape d'injection de l'écoulement de gaz contenant du CO₂ à l'intérieur du puits (4) sans paroi de séparation défléchissante selon la revendication 1, au moyen du dispositif d'injection (5) situé au niveau de la base dudit puits adjacent à la paroi (6) opposée à l'entrée (2) du courant d'eau et à une hauteur telle que le côté supérieur du dispositif d'injection (5) est à une distance entre 0,5 et 1,5 m du fond du canal d'écoulement (1), de maintien du débit de gaz dans un écoulement volumétrique de CO₂ entre 0,008 et 0,025 v/v/mn et d'une vitesse de circulation de liquide entre 10 et 50 cm/s, jusqu'à l'obtention d'un rapport de débit liquide:gaz supérieur à 10.

13. Procédé de carbonatation selon la revendication précédente, dans lequel le rapport liquide:gaz est entre 20 et 30 cm/sg.

14. Utilisation du système de carbonatation décrit dans l'une quelconque des revendications 1 à 11 pour le traitement d'eau usée au moyen de systèmes de micro-algues-bactéries mélangées.

15. Utilisation du système de carbonatation décrit dans l'une quelconque des revendications 1 à 11 pour la production d'algues à des fins énergétique et alimentaire ou pour la production de composés à haute énergie.

16. Utilisation du système de carbonatation décrit dans l'une quelconque des revendications 1 à 11 pour la purification de gaz industriels au moyen de l'absorption des polluants présents dans les gaz et d'une fixation biologique subséquente au moyen de la production de micro-algues.
